# EUROPEAN PATENT SPECIFICATION

(11) **EP 2 084 191 B1**
(45) Date of publication and mention of the grant of the patent: **23.10.2013**
(21) Application number: 07728174.9
(22) Date of filing: 17.04.2007
(51) Int. Cl.: C08B 37/14, A61K 8/73, C11D 1/00

(54) **PROCEDURE FOR THE PREPARATION OF GLYOXALATED CATIONIC GUAR**
VERFAHREN ZUR HERSTELLUNG VON GLYOXALIERTEM KATIONISCHEM GUAR
PROCÉDURE DE PRÉPARATION DE GUAR CATIONIQUE GLYOXALATÉ

(30) Priority: 17.11.2006 IT VA20060068
(43) Date of publication of application: 05.08.2009
(73) Proprietor: Lamberti Spa, 21041 Albizzate (VA) (IT)
(72) Inventor: BALDARO, Eva, 20127 Milano (IT); TENCONI, Mauro, 21045 Gazzada (VA) (IT); LI BASSI, Giuseppe, 21026 Gavirate (VA) (IT)
(86) International application number: PCT/EP2007/053709
(87) International publication number: WO 2008/058768

(56) References cited:
- WO-A-03/078473
- CA-A1- 2 063 365
- US-A- 3 350 386

## Description

### Technical Field

The present invention relates to a procedure for the preparation of glyoxalated purified cationic guar which is soluble at basic pH and suitable for the use in the cosmetic field and in household cleaning products.

The cationic guar obtained by the procedure of the present invention has the technologically useful property to be soluble in water only at basic pH, and therefore to develop viscosity only in aqueous solutions having pH greater than 7; moreover it possesses a degree of purity that renders it suitable for the cosmetic field.

Cationic polysaccharides are derivatives of natural origins, very used as industrial additives due to their conditioning properties (i.e. they improve the characteristics of the substrate, generally paper, skin, hair or fabric, to which they are applied on).

This characteristic renders them industrially useful for the preparation of shampoos, hair conditioners, creams, personal or household care detergents and for softeners that confer a soft touch and antistatic properties to fabrics (see as an example Conditioning Agents for Hair & Skin, Ed. R. Schueller and P. Romanowski, Marcel Dekker Inc, NY, 1999).

Beside their conditioning power, the capability of these polysaccharides to thicken and regulate the rheology of the solutions in which they are dissolved is industrially useful.

In particular, cationic polygalactomannans, and among these cationic derivatives of guar gum, have shown optimal results in improving the wet and dry combability of hairs washed with a shampoo formulated therewith.

It is well known that, in cosmetic formulations, the presence of materials not expressly added and controlled, not predicted and variable from batch to batch, even if in minimal amounts, can create problems during the preparation of the formulation, such as phase separation and variations of the viscosity.

Moreover, during the last few years, particular attention has been placed to the toxicity of raw materials used for the fabrication of cosmetic products. The EC Directive 76/768/EEC and following modifications, as an example, limits or prohibits the presence of certain substances in raw materials for cosmetics, with the aim of safeguarding the consumer health.

In particular, the above cited directive prohibits the presence in cosmetic products of several substances, listed in the Attachment II, and classified as carcinogenic, mutagenic or toxic for reproduction, pertaining to categories CMR 1, CMR 2 or CMR 3, according to the EEC Directive 67/548/EEC; the presence of other substances, not necessarily present as ingredients, but only as impurities deriving from the process of production of the raw materials, is subjected to amount limitations according to the same EEC Directive 76/768/EEC (substances listed in Attachment III).

It is therefore of fundamental importance that, for the use in cosmetic formulations and in formulations that come to direct contact with skin, such as in household cleaning products, the cationic derivatives of guar are as much as possible devoid of impurities, both for the consumer's health and for technological problems connected with the production of cosmetics.

Furthermore, for the use in cosmetic formulations or in household detergents having a pH greater than 7, it is essential that the guar cationic derivatives are able to show their conditioning and thickening capacity at basic pH.

### Background Art

Cationic guar derivatives are known since the early '70s, when their use is cited in the production of waterproof paper (see US 3,589,978).

The first use of cationic guar derivatives in cosmetics goes back to 1977, when a cationic derivative of guar was used in the preparation of a so-called "two in one" shampoo, having hair conditioning characteristics beside the normal detergent power (see US 4,061,602).

The cationic guar derivative used in cosmetic is known with the INCI name of Guar Hydroxypropyltrimonium Chloride and, chemically, is guar 2-hydroxy-3-(trimethylammonio)propyl ether chloride.

Its synthesis, described as an example in the cited patent

US 3,589,978, requires the reaction of 2,3-epoxypropyl trimethylammonium chloride or (3-chloro-2-hydroxypropyl) trimethylammonium chloride on the hydroxyl groups of guar, in the presence of basic catalysts (as an example sodium hydroxide).

The reagent 2,3-epoxypropyl trimethyl ammonium chloride is classified as a carcinogenic substance CMR 2; the reagent 3-chloro-2-hydroxypropyl trimethylammonium chloride, even if less toxic than the corresponding epoxide, is classified as CMR 3 and in alkaline medium is converted into 2,3-epoxypropyl trimethyl ammonium chloride.

According to what is described in US 3,589,978, the reaction can be carried out in a solvent such as isopropanol, methanol, ethanol and tert-butanol, at temperatures between 30 and 60°C; in the Example A of the same patent, at the end of the reaction and after neutralisation of the alkali excess, the product is dried, milled and washed with methanol, in the attempt to eliminate the un-reacted quaternary reagent.

Nothing is reported in US 3,589,978 on the residual quantities of the cationic reagent (2,3-epoxypropyl trimethyl ammonium chloride) in the final product; but we can observe that methanol is itself a toxic product.

In US 4,031,307 the preparation of cationic derivatives of guar in a biphasic system is described, by reacting solid guar with a cationising reagent in a mixture of water and water soluble solvent that contains the basic catalyst; after the reaction, the obtained product is separated by centrifugation or filtration and preferably purified by means of a first washing with the water-solvent mixture used in synthesis and with a second washing with a more anhydrous form of the same solvent.

In US 2001051143, the preparation of guar cationic derivatives comprising at the end of the reaction a first washing with 85% by weight aqueous isopropanol and a second washing with pure isopropanol is described; in US 2001051140 the preparation of guar cationic derivative comprising, at the end of the reaction, two washings with 85% aqueous isopropanol is described.

In all these publications, neither the type nor the amount of impurities present in the obtained cationic guar derivative is mentioned, impurities that will become part of the final cosmetic product in case the cationic guar derivative is used as a cosmetic raw material.

Moreover, the washings of cationic guar derivatives carried out with water and solvent, each one necessarily involving the removal of the used solvent mixture (by filtration, for example), is economically burdensome, because of the length of time needed, of the waste treatment, of the decrease of the process yield.

The greatest part of cationic guar derivatives currently on the market, in order to obviate to the difficulty and to the burden connected with the purification by washings with water and solvents, are purified by washing the product, previously cross-linked with borates, with water only, as described for example in CA 2,023,324, where in the reaction phase borax is added; the cationic guar derivatives so obtained contain small amounts of boron (borated guars).

The aim of the cross-linking with boric acid is to form bonds, through the borate anion, between the polysaccharides chains, that render the product insoluble in water.

These bonds are stable at basic pH, and therefore in such conditions the product can be washed from by-products.

In acidic conditions the bonds with the borate are removed, the product is soluble and can perform its thickening and conditioning properties.

The reaction with borates is reversible with pH changes; therefore, even if the product is pre-solubilised at acidic pH, bringing it back to alkaline conditions may cause a change in the viscosity of the formulate, varying as a consequence the quality of the product.

The limit of borated cationic guar derivatives is their exclusive applicability to products to be used at acidic or slightly acidic pH, because products treated with borates are not soluble at pH greater than 7.

Furthermore, it must be observed that boric acid derivatives are classified as substances toxic for reproduction of category CMR 2.

Hair dyes represent one of the sector of the home & personal care in the greatest expansion; they are generally formulated at pH higher than 8 and therefore it is not possible to formulate them with cationic guar derivatives purified by cross-linking with boric acid.

The preparation of solid soap bars, generally having pH higher than 7.5 in aqueous solution and normally containing softening and conditioning agents for the skin, represent another field of great interest for the use of non borated cationic guar derivatives.

The preparation of depilatory creams and the production of fabric powder detergents, having generally basic pH and possibly advantageously containing a co-formulating agent having skin protective function, are further formulations in which non-borated cationic guar derivatives can be used.

In EP 1,490,408 B1 the Applicant described purified non-cationic guar derivatives, containing glyoxal, which are soluble at basic pH, have high thickening properties and are useful as additives for the building industry.

The glyoxalation procedure (cross-linking with glyoxal) of natural polymers containing hydroxyl functions has been known for a long time: in US 3,297,583 a procedure is described to obtain macromolecular substances carrying a plurality of hydroxyl groups fast dissolving in water, by means of a treatment with glyoxal and pH regulation of the water solution between 7 and 10.

US 3,350,386 describes a method to purify non-cationic guar derivatives by reaction with glyoxal, drying, washing, and further drying.

CA 2,063,365 describes a procedure to modify guar splits and its derivatives with glyoxal; the splits constitute the cotyledon of the guar seed and are obtained from the guar seed after elimination of the external husk and the germ.

The Applicant has now found a procedure for the preparation of glyoxalated purified cationic guar, soluble at basic pH, free from boron and with a reduced content of other impurities that can compromise the stability or the characteristics of the cosmetic formulations themself.

### Disclosure of Invention

It is therefore a fundamental object of the present invention a procedure for the preparation of cationic guar having DS comprised between 0.01 and 3 comprising the following steps: a) 100 parts by weight of guar flour are reacted with 3-chloro-2-hydroxypropyl trimethylammonium chloride and sodium hydroxide, in from 5 to 500 parts of a water and alcohol mixture containing from 20 to 50% by weight of water; b) the mixture is acidified to a pH between 4 and 6; c) from 2.2 to 3 parts by weight of glyoxal are added, and the reaction is stirred for approximately 30 minutes; d) from 300 to 1200 parts by weight of ambient temperature water are added and mixed for 10 to 90 minutes; e) the mixture is filtered under vacuum and the product dried to obtain glyoxalated purified cationic guar.

The glyoxalated purified cationic guar obtained by procedure of the invention is a further object of the present invention.

The guar flour utilisable for the invention is any commercially available guar flour, preferably containing 10% by weight maximum of water.

With the expression "cationic guar" in the present text we mean guar 2-hydroxy-3-(trimethylammonio)propyl ether chloride.

In order to obtain a cationic guar having a degree of substitution (DS) comprised between 0.01 and 3 in step a) the reaction is carried out, under stirring, using from 2 to 600 parts by weight of 3-chloro-2-hydroxypropyltrimethylammonium chloride, and from 0.4 to 160 parts by weight of sodium hydroxide (or equivalent amount of other strong base).

In the present text, with the expression "degree of substitution" (DS) we mean the substitution of the cationic group on the hydroxyl groups of guar measured by means of ¹H-NMR.

According to a preferred realisation of the present invention, in step a) of the reaction from 10 to 100 parts by weight of 3-chloro-2-hydroxypropyltrimethylammonium chloride and from 2 to 27 parts by weight of sodium hydroxyde are used, obtaining at the end of the preparation a cationic guar having a DS comprised between 0.05 and 0.5; this degree of substitution permits to obtain the best conditioning performances.

Usually, according to what is well known in the field, in step a) the reaction is carried out at a temperature comprised between 40 and 80 °C for 0.5-4 hours; the acidification of step b) and the reaction of step c) are carried out at a temperature comprised between 20 and 45°C; the drying of step e) is carried out at a temperature comprised between 60 and 90 °C and after the filtration of step e) the purified cationic guar is milled.

The alcohol useful for the procedure of the invention is ethanol, isopropanol, or mixtures thereof.

Preferably in step a) of the procedure from 50 to 200 parts by weight of water and alcohol mixture are used.

The washing with water of steps d) and e) permits to eliminate at least 90% of the impurities (water and alcohol residues excluded) and to obtain a glyoxalated purified cationic guar.

The product obtained by means of the procedure of the present invention is insoluble at pH lower than 7 and quickly and completely soluble at pH higher than 8.

The glyoxalated and purified guar 2-hydroxy-3-(trimethylammonio) propyl ether chloride of the invention contains from 0.3 to 1.5% by weight, preferably between 0.3 and 0.8% by weight of glyoxal, it is soluble at pH higher than 8 and has a degree of cationic substitution (DS) comprised between 0.01 and 3.0, preferably between 0.05 and 0.5.

The glyoxal contained in the glyoxalated purified cationic guar of the invention does not influence in any way its applicability in the field of home & personal care.

The cationic guar in fact shows its conditioning and viscosifying characteristics when dosed at rather low concentrations, typically comprised between 0.01 % and 0.5% of the formulated cosmetic; glyoxal, which is possibly present in the formulated cosmetic when the cationic guar of the invention is used as an ingredient, is therefore widely under the 100 ppm limit of the current legislation.

The cationic guar of the invention is free from boron, toxic solvents, 3-chloro-2-hydroxypropyltrimethylammonium chloride and has minimum contents of 2,3-dihydroxypropyl trimethylammonium chloride (possibly formed by the reaction of 3-chloro-2-hydroxypropyltrimethylammonium chloride with water during the cationising reaction).

An advantage of the procedure according the invention is that it allows to obtain the purified cationic guar with high yields, due to the fact that at the pH of washing the product is completely insoluble in water.

The use of the reported amount of glyoxal is essential for this purpose, because it guarantees the insolubility of the product during the washing of step d), and a fast and complete dissolution of the glyoxalated purified cationic guar at pH higher than 8.

The determination of the cationising reagent residue and its correlated glycol is carried out by means of ion exchange chromatography, by the use of a cationic exchange column and elution with methanesulphonic acid solution.

With the expression "free from 3-chloro-2-hydroxypropyl trimethylammonium chloride" we mean that in the cationic guar of the invention the concentration of from 3-chloro-2-hydroxypropyl trimethylammonium chloride is below the detection limit of the above described method (in this case below 0.15%).

The product obtained by means of the procedure of the invention can be used in the most different cosmetic formulations, where its capability to bind through its positive charges to substrates having weak negative charges, together with its capability to thicken and to regulate the rheology of water solutions are exploited.

A further advantage of the procedure of the invention is that the cationic guar so obtained contains less than 2% by weight of inorganic salts (amount determined by calcination at 700°C) and in particular, less than 1% by weight of sodium chloride, whose presence, as it is well known, influences the effectiveness of the thickeners commonly used in the cosmetic field.

It is believed that in the procedure of the invention the use of guar in the form of flour is fundamental in order to obtain the characteristics of purity which are typical of the cationic guar of the invention.

The cationic guar of the invention is also useful in other industrial fields, where the purity characteristics of the product are of particular importance, as for example in the house care.

EXAMPLE 1

In a 5 litres stirred reactor, 800 g of guar flour are loaded at room temperature, the reaction atmosphere is made inert by means of vacuum/nitrogen washings, and under vigorous stirring 50 g of sodium hydroxide dissolved in 450 g of a 1/9 water/isopropanol solution are added. The stirring is continued for 30 minutes at a temperature of 50-60°C; 224 g of 3-cloro-2-hydroxypropyl trimethylammonium chloride 85% diluted in 100 g of water are added. After 2 hours at the same temperature the reaction is cooled off to 40°C and neutralised by addition of acetic acid to pH about 5.

46 g of glyoxal (40% in water) are added, and left to react for about 1 hour keeping the temperature at about 40°C.

The so obtained reaction mixture (glyoxalated raw cationic guar), is dispersed in 2900 g of tap water at pH below 7, left under stirring for 10 minutes, then filtered under vacuum (0.4-0.5 atm) on a fabric filter.

The filtered product is washed with 1900 g of tap water at pH below 7, adding this last washing directly on the wet product present on the filter and applying vacuum.

The product (purified cationic guar 2.3% glyoxalated) is dried on a fluid bed drier using hot air until the humidity content is about 3% by weight, milled and analysed.

The bonded glyoxal content is determined by reaction with 2-hydrazono-2,3-dihydro-3-methylbenzothiazole chlorohydrate, according to the method described in "Kunststoffe im Lebensmittelverkehr" Ed. Carl Heymanns Verlag KG, 1999, page 228-231 and is 0.37% by weight.

EXAMPLE 2

In a 5 litres stirred reactor, 800 g of guar flour are loaded at room temperature, the reaction atmosphere is made inert by means of vacuum/nitrogen washings, then under vigorous stirring 120 g of sodium hydroxide dissolved in 500 g of a 1/9 water/isopropanol solution are added. The stirring is carried out for 30 minutes at a temperature of 50-60°C and 560 g of 3-cloro-2-hydroxypropyl trimethylammonium chloride 85% diluted in 100 g of water are added. After 2 hours at the same temperature the reaction is cooled off to 40°C and the reaction neutralised by means of acetic acid to pH about 5.

46 g of glyoxal (40% in water) are added, and left to react for about 1 hour keeping the temperature at about 40°C.

The so obtained reaction mixture (glyoxalated raw cationic guar), is washed and dried as in Example 1 and then analysed.

The glyoxal content is 0.50%

EXAMPLE 3 (comparative)

In a 5 litres stirred reactor, 800 g of guar flour and 2 g of borax are loaded at room temperature, the reaction atmosphere is made inert by means of vacuum/nitrogen washings, then under good stirring 50 g of sodium hydroxide dissolved in 450 g of a 1/9 water/iso-propanol solution are added. The stirring is carried out for 30 minutes at a temperature of 50-60°C; 224 g of 3-cloro-2-hydroxypropyl trimethylammonium chloride 85% diluted in 100 g of water are added. After 2 hours at the same temperature the reaction is cooled off.

The so obtained reaction mixture (borated raw cationic guar) is dispersed in 2900 g of tap water, left under stirring for 10 minutes and filtered under vacuum (0.4-0.5 atm) on a fabric filter. The filtered product is then washed with 1900 g of tap water, adding this last washing directly on the wet product present on the filter and applying the vacuum.

The product (purified cationic guar borated) is dried on a fluid bed drier using hot air until a humidity content of about 3% by weight, milled and analysed.

The bonded boron content is determined by microanalysis and is reported as boric acid content.

EXAMPLE 4 (comparative)

In a 5 litres stirred reactor, 800 g of guar flour are loaded at room temperature, the reaction atmosphere is made inert by means of vacuum/nitrogen washings, then under good stirring 50 g of sodium hydroxide dissolved in 450 g of a 1/9 water/isopropanol solution are added. The stirring is carried out for 30 minutes at a temperature of 50-60°C; 224 g of 3-cloro-2-hydroxypropyl trimethylammonium chloride 85% diluted in 100 g of water are added. After 2 hours at the same temperature the reaction is cooled off and the reaction neutralised by with acetic acid. The so obtained product (raw cationic guar), is dried on a fluid bed drier using hot air until a humidity content of about 3% by weight, milled and analysed.

In Table 1 the values of the analytical determinations performed on the products prepared in the Examples 1-4 are reported.

**Table 1**

| Ex. | Guar product | DS | Ashes % | Chloridrine* | Glycol* | Boric Acid |
|---|---|---|---|---|---|---|
| 1 | Purified cationic guar glyoxalated | 0.15 | 0.8% | absent | 0.3% | - |
| 2 | Purified cationic guar glyoxalated | 0.3 | 1.2% | absent | 0.3% | - |
| 3 | Purified cationic guar borated | 0.15 | 1.0% | absent | 0.3% | 0.2% |
| 4 | Raw cationic guar | 0.3 | 7.0% | 0.39% | 2.66% | - |

*Chloridrine = 3-chloro-2-hydroxypropyl trimethylammonium chloride

Glycol = 2,3-dihydroxypropyl trimethylammonium chloride

## Claims

1. Procedure for the preparation of cationic guar having DS comprised between 0.01 and 3 comprising the following steps: a) 100 parts by weight of guar flour are reacted with 3-chloro-2-hydroxypropyl trimethylammonium chloride and sodium hydroxide in from 5 to 500 parts of a water and alcohol mixture containing from 20 to 50% by weight of water; b) the mixture is acidified to a pH between 4 and 6; c) from 2.2 to 3 parts by weight of glyoxal are added, and the reaction is stirred for approximately 30 minutes; d) from 300 to 1200 parts by weight of ambient temperature water are added and mixed for 10 to 90 minutes; e) the mixture is filtered under vacuum and the product dried to obtain glyoxalated purified cationic guar.

2. Procedure according to claim 1., where in step a) the reaction is carried out, under stirring, using from 2 to 600 parts by weight of 3-chloro-2-hydroxypropyltrimethylammonium chloride and from 0.4 to 160 parts by weight of sodium hydroxide, at temperature comprised between 40 and 80 °C for 0.5-4 hours; the acidification of step b) and the reaction of step c) are performed at temperature comprised between 20 and 45°C; the drying of step e) is performed at temperature comprised between 60 and 90 °C.

3. Procedure according to claim 1. or 2., wherein the reaction of step a) is performed in from 50 to 200 parts by weight of the water and alcohol mixture.

4. Procedure according to claim 3., where the alcohol is ethanol or isopropanol, or mixtures thereof.

5. Procedure according to claim 4., where in step a) from 10 to 100 parts by weight of 3-chloro-2-hydroxypropyltrimethylammonium chloride and from 2 to 27 parts by weight of sodium hydroxide are used.

6. Procedure according to claim 5. where during the filtration of step e), the product is washed with from 100 to 400 parts by weight of water.

7. Procedure according claim 6. where after the filtration of step e) the purified cationic guar is milled.

8. Guar 2-hydroxy-3-(trimethylammonium)propyl ether chloride having a cationic degree of substitution (DS) comprised between 0.01 and 3.0, **characterised by** the fact that it contains from 0.3 to 1.5% by weight of glyoxal, it is soluble at pH>8 and insoluble at pH < 7, is boron free and free from 3-chloro-2-hydroxypropyltrimethylammonium chloride and contains less than 2% by weight of inorganic salts and, in particular, less than 1% by weight of sodium chloride.

9. Use of guar 2-hydroxy-3-(trimethylammonium)propyl ether chloride according to claim 8 in the preparation of cosmetic formulations.

10. Use of guar 2-hydroxy-3-(trimethylammonium)propyl ether chloride according to claim 8 in the preparation of household cleaning products.

## Patentansprüche

1. Verfahren zur Herstellung von kationischem Guar mit einem SG, der zwischen 0,01 und 3 liegt, wobei das Verfahren die folgenden Schritte umfasst: a) 100 Gewichtsteile Guarmehl werden mit 3-Chlor-2-hydroxypropyltrimethylammoniumchlorid und Natriumhydroxid in 5 bis 500 Teile eines. Wasser/Alkohol-Gemischs, das 20 bis 50 Gew.-% Wasser enthält, reagieren gelassen; b) das Gemisch wird auf einen pH-Wert zwischen 4 und 6 angesäuert; c) 2,2 bis 3 Gewichtsteile Glyoxal werden zugegeben und das Reaktionsgemisch wird für ungefähr 30 Minuten gerührt; d) 300 bis 1200 Gewichtsteile Wasser mit Umgebungstemperatur werden zugegeben und es wird für 10 bis 90 Minuten gemischt; e) das Gemisch wird unter Vakuum filtriert und das Produkt wird getrocknet, um glyoxyliertes aufgereinigtes kationisches Guar zu erhalten.

2. Verfahren nach Anspruch 1, wobei in Schritt a) die Reaktion unter Rühren unter Verwendung von 2 bis 600 Gewichtsteilen 3-Chlor-2-hydroxypropyltrimethylammoniumchlorid und 0,4 bis 160 Gewichtsteilen Natriumhydroxid bei einer Temperatur, die zwischen 40 und 80 °C liegt, für 0,5 - 4 Stunden durchgeführt wird; die Ansäuerung von Schritt b) und die Reaktion von Schritt c) bei einer Temperatur durchgeführt werden, die zwischen 20 und 45 °C liegt; die Trocknung von Schritt e) bei einer Temperatur durchgeführt wird, die zwischen 60 und 90 °C liegt.

3. Verfahren nach Anspruch 1 oder 2, wobei die Reaktion von Schritt a) in 50 bis 200 Gewichtsteilen des Wasser/Alkohol-Gemischs durchgeführt wird.

4. Verfahren nach Anspruch 3, wobei es sich bei dem Alkohol um Ethanol oder Isopropanol oder Gemische davon handelt.

5. Verfahren nach Anspruch 4, wobei in Schritt a) 10 bis 100 Gewichtsteile 3-Chlor-2-hydroxypropyltrimethylammoniumchlorid und 2 bis 27 Gewichtsteile Natriumhydroxid verwendet werden.

6. Verfahren nach Anspruch 5, wobei während der Filtration von Schritt e) das Produkt mit 100 bis 400 Gewichtsteilen Wasser gewaschen wird.

7. Verfahren nach Anspruch 6, wobei nach der Filtration von Schritt e) das aufgereinigte kationische Guar gemahlen wird.

8. Guar-2-Hydroxy-3-(trimethylammonium)propyletherchlorid mit einem kationischen Substitutionsgrad (SG), der zwischen 0,01 und 3,0 liegt, **gekennzeichnet durch** die Tatsache, dass es 0,3 bis 1,5 Gew.-% Glyoxal enthält, bei einem pH-Wert > 8 löslich und bei einem pH-Wert < 7 unlöslich ist, borfrei ist und frei von 3-Chlor-2-hydroxypropyltrimethylammoniumchlorid ist und weniger als 2 Gew.-% anorganische Salze und insbesondere weniger als 1 Gew.-% Natriumchlorid enthält.

9. Verwendung von Guar-2-Hydroxy-3-(trimethylammonium)propyletherchlorid nach Anspruch 8 bei der Herstellung von kosmetischen Formulierungen.

10. Verwendung von Guar-2-Hydroxy-3-(trimethylammonium)propyletherchlorid nach Anspruch 8 bei der Herstellung von Haushaltsreinigungsprodukten.

## Revendications

1. Procédé de préparation de guar cationique possédant un DS compris entre 0,01 et 3 comprenant les étapes suivantes : a) 100 parts par poids de farine de guar réagissent avec un chlorure de 3-chloro-2-hydroxypropyl triméthylammonium et un hydroxyde de sodium, et de 5 à 500 parts par poids d'eau et d'un mélange d'alcool contenant de 20 à 50% en poids d'eau, (b) le mélange est acidifié à un pH compris entre 4 et 6, (c) de 2,2 à 3 parts par poids de glyoxal sont ajoutées, et la réaction est mélangée pendant approximativement 30 minutes, (d) de 300 à 1200 parts par poids d'eau à température ambiante sont ajoutées et mélangées pendant 10 à 90 minutes, (e) le mélange est filtré sous vide et le produit est séché afin d'obtenir un guar cationique glyoxalaté purifié.

2. Procédé selon la revendication 1, dans lequel dans l'étape a) la réaction est réalisée, sous agitation, en utilisant de 2 à 600 parts par poids de chlorure de 3-chloro-2-hydroxypropyl triméthylammonium et de 0,4 à 160 parts par poids d'hydroxyde de sodium, à une température comprise entre 40 et 80°C pendant 0,5 à 4 heures ; l'acidification de l'étape b) et la réaction de l'étape c) sont réalisées à une température comprise entre 20 et 45°C ; le séchage de l'étape e) est réalisé à une température comprise entre 60 et 90°C.

3. Procédé selon la revendication 1 ou 2, dans lequel la réaction de l'étape a) est réalisée en utilisant de 50 à 200 parts par poids d'eau et mélange d'alcool.

4. Procédé selon la revendication 3, dans lequel l'alcool est de l'éthanol ou de l'isopropanol, ou des mélanges de ceux-ci.

5. Procédé selon la revendication 4, dans lequel dans l'étape a) de 10 à 100 parts par poids de chlorure de 3-chloro-2-hydroxypropyl triméthylammonium et de 2 à 27 parts par poids d'hydroxyde de sodium sont utilisées.

6. Procédé selon la revendication 5, dans lequel durant la filtration de l'étape e), le produit est lavé avec 100 à 400 parts par poids d'eau.

7. Procédé selon la revendication 6, dans lequel après la filtration de l'étape e) le guar cationique purifié est broyé.

8. Guar, ether 2-hydroxy-3-(trimethylammonio) propylique, chlorure possédant un degré de substitution cationique (DS) compris entre 0,01 et 3, **caractérisé par le fait qu'**il contient de 0,3 à 1,5% par poids de glyoxal, qu'il est soluble avec un pH supérieur à 8 et insoluble avec un pH inférieur à 7, est exempt de bore et de chlorure de 3-chloro-2-hydroxypropyl triméthylammonium et contient moins de 2% par poids de sels inorganiques et, en particulier, moins de 1% par poids de chlorure de sodium.

9. Utilisation de Guar, ether 2-hydroxy-3-(trimethylammonio) propylique, chlorure selon la revendication 8 dans la préparation de produits cosmétiques.

10. Utilisation de Guar, ether 2-hydroxy-3-(trimethylammonio) propylique, chlorure selon la revendication 8 dans la préparation de produits d'entretien.
